# FASCICULE DE BREVET EUROPEEN

(11) **EP 2 906 338 B1**
(45) Date de publication et mention de la délivrance du brevet: **11.12.2019**
(21) Numéro de dépôt: 13786689.3
(22) Date de dépôt: 08.10.2013
(51) Int. Cl.: B01J 13/10, A61K 8/11, A61K 9/50, B01J 13/22, C09B 67/02, C11D 3/50

(54) **PROCEDE D'ENCAPSULATION PAR COACERVATION NE METTANT PAS EN OEUVRE DE RETICULANT TOXIQUE**
KOAZERVATIONSVERKAPSELUNGSVERFAHREN OHNE VERWENDUNG VON TOXISCHEN VERNETZERN
COACERVATION ENCAPSULATION METHOD THAT DOES NOT INVOLVE THE USE OF TOXIC CROSS-LINKING AGENTS

(30) Priorité: 09.10.2012 FR 1259631
(43) Date de publication de la demande: 19.08.2015
(73) Titulaire: Société d'Exploitation de Produits pour les Industries Chimiques SEPPIC, 75321 Paris cedex 07 (FR)
(72) Inventeur: OLLAGNIER, Jean-Noël, F-81100 Castres (FR)
(74) Mandataire: Grout de Beaufort, François-Xavier
(86) Numéro de dépôt international: PCT/FR2013/052385
(87) Numéro de publication internationale: WO 2014/057202

(56) Documents cités:
- WO-A2-2011/117738
- US-A1- 2006 144 412
- US-A1- 2007 269 566

## Description

La présente invention concerne le procédé d'obtention de capsules à double parois obtenues par coacervation sans la mise en œuvre d'agent réticulant.

La coacervation décrit le phénomène de désolvatation de macromolécules, telles que des polymères, conduisant à une séparation de phases au sein d'une solution. La coacervation simple se rapporte aux procédés faisant intervenir la désolvatation d'un seul polymère par l'un des facteurs suivants : abaissement de température, addition d'un non-solvant, addition d'électrolytes, addition d'un deuxième polymère incompatible. Lorsque la désolvatation simultanée de deux polyélectrolytes hydrosolubles portant des charges opposées est provoquée par une modification de pH du milieu aqueux, on parle de coacervation complexe.

La coacervation complexe est une technique d'encapsulation bien connue et industrialisée depuis les années 50. Elle permet d'encapsuler des ingrédients insolubles dans l'eau. Le brevet US 2 800 457 du 23 juillet 1957 décrit par exemple le procédé d'encapsulation d'huiles dans un coacervat de deux polymères organiques : la gélatine et la gomme arabique. Le brevet canadien CA88263 du 5 octobre 1971 décrit un procédé similaire utilisant un polymère organique et un polymère minéral.

Le procédé de fabrication de microcapsules par cette technique se déroule généralement en cinq étapes successives.

Dans un premier temps, le produit à encapsuler (sous forme liquide ou solide, pur ou en solution huileuse) est dispersé dans une solution aqueuse contenant deux polymères de charges opposées (étape a).

Dans un deuxième temps, la coacervation est induite par un ajustement du pH de la solution, de façon que les charges positives du premier polymère équilibrent les charges négatives du second (étape b). L'attraction électrostatique des deux polyélectrolytes provoque l'apparition d'un coacervat mixte.

Dans un troisième temps, les gouttelettes de coacervat formées viennent s'adsorber (étape c) à la surface de la matière active à encapsuler et former un enrobage continu (étape d).

Dans la quatrième étape, cet enrobage est consolidé par réticulation (étape e) des macromolécules constitutives du coacervat pour former des microcapsules stables.

Finalement, les microcapsules sont séparées du milieu réactionnel par décantation et filtration, avant de subir des opérations de lavage ou de purification pour éliminer les produits n'ayant pas réagi, en particulier l'excès d'agents de réticulation, et éventuellement des opérations de séchage.

Parmi les macromolécules ou polymères organiques à caractère cationique utilisables dans la technologie de coacervation, on peut citer de manière non limitative les protéines animales comme la gélatine de porc ou de poisson, l'albumine, les protéines végétales issues par exemple du soja, de la pomme de terre ou du blé, le chitosan et ses dérivés, les polymères synthétiques issus de la combinaison d'amino acides comme la polylysine ou encore les polymères d'origine végétale comme la gomme guar et ses dérivés.

Parmi les polymères organiques anioniques pouvant être mis en œuvre se trouvent les polymères naturels comme la gomme arabique, les alginates, les carraghénates, les dérivés de la cellulose comme la carboxyméthyle cellulose, les dérivés d'amidon comme le carboxyméthyle amidon, ou les polymères synthétiques comme les polymères acryliques, méthacryliques, polylactiques, polyglycoliques ou leurs combinaisons.

Les ingrédients encapsulés peuvent être des principes actifs cosmétiques comme des filtres solaires, des huiles essentielles, les vitamines A, D, E ou leurs dérivés, des lipoaminoacides.

Pour obtenir des capsules suffisamment résistantes mécaniquement, la réticulation de l'étape (e) est indispensable. Cette opération met en jeu un agent réticulant. Parmi les agents réticulants les plus efficaces, on peut citer le formaldéhyde ou le glutaraldéhyde. D'autres réticulants ont également été proposés, comme les carbodiimides, les isocyanates (HDI ou Hexaméthylène diisocyanate, TDI ou Toluène Di Isocyanate, IPDI ou Isopropyl Di Isocyanate), les proanthocyanidines... Tous ces réticulants ont soit une toxicité non négligeable ou sont instables en milieu aqueux et doivent être utilisés dans des conditions qui compliquent l'étape de réticulation. D'autres auteurs ont décrit des procédés de réticulation avec des enzymes telles que les transglutaminases ou la génépine. Les coûts de ces produits sont tels que seules quelques applications à très fortes valeurs ajoutées peuvent être envisagées.

Un agent réticulant est un composé chimique qui permet de relier une chaîne polymérique à une autre par la formation de liaisons covalentes. Dans l'état de la technique, il s'agit notamment d'une réaction entre les fonctions aldéhydes du réticulant et les fonctions amines résiduelles des protéines, en particulier avec les fonctions amines des motifs lysine pour former des liaisons covalentes -N=CH-.

Le glutaraldéhyde est l'agent réticulant le plus souvent mis en œuvre. Il est efficace et peu onéreux. Mais il doit être utilisé à des doses élevées, voisines de 1 à 5 moles/kg de gélatine (soit 100 à 500g/kg de protéine) et présente une toxicité non négligeable tant pour le manipulateur que pour l'utilisateur. L'élimination du glutaraldéhyde en excès est indispensable, notamment pour toutes les applications pharmaceutiques, alimentaires ou cosmétiques ; elle nécessite de nombreux lavages successifs qui sont consommateurs d'eau et de temps pour obtenir des microcapsules contenant un taux résiduel de glutaraldéhyde acceptable, inférieur à une centaine de ppm.

Un premier problème à résoudre pour les inventeurs de la présente invention est donc de produire des microcapsules suffisamment résistantes par une technique de coacervation complexe qui n'utilise pas d'agent de réticulation.

Compte-tenu du principe même du procédé de coacervation, seuls des actifs lipophiles, insolubles dans l'eau peuvent être incorporés dans les microcapsules obtenues par cette technologie. Cela constitue indubitablement une limitation du procédé alors que de très nombreux ingrédients hydrosolubles doivent être également incorporés dans des microcapsules.

Un objet de l'invention est donc de développer une technique de coacervation améliorée, sans mise en œuvre de réticulants toxiques ou onéreux, et permettant d'encapsuler à la fois des ingrédients solubles et insolubles dans l'eau.

Pour encapsuler des ingrédients hydrosolubles, d'autres techniques ont été décrites telles que, par exemple, la granulation au moyen de polymères hydrophiles, l'émulsification dans des huiles sous forme d'émulsions de type eau dans huile, l'absorption sur des résines échangeuses d'ions pour former des résinâtes. Une des technologies les plus utilisées pour encapsuler des ingrédients hydrosolubles reste l'incorporation dans des microbilles de polymères hydrosolubles comme les chitosans, les alcools polyvinyliques ou les alginates. Ces derniers sont mis en œuvre dans de nombreuses applications pharmaceutiques ou alimentaires pour obtenir des microbilles par un procédé de coacervation simple, aussi appelé dripping. A titre d'exemple, on trouvera la description d'un tel procédé d'encapsulation de cellules dans la demande de brevet WO9109119 du 27 juin 1991.

Le dripping consiste à préparer une solution aqueuse contenant l'ingrédient hydrosoluble à encapsuler et un polymère tel que l'alginate de sodium. Cette solution est mise sous pression à travers des buses calibrées pour former des gouttes recueillies dans une solution aqueuse de sels divalents comme le chlorure de calcium, de magnésium ou de manganèse. Les ions calcium réagissent avec l'alginate de sodium pour former immédiatement des billes solides, insolubles, d'alginate de calcium. Les billes obtenues sont séparées par filtration ou tamisage puis généralement lavées à l'eau pour éliminer le chlorure de calcium en excès.

WO 2011/117738 A2 décrit un procédé de préparation de particules encapsulées comprenant les étapes suivantes: dispersion d'un arôme hydrophobe dans de l'eau comprenant protéine de soja et kappa-carraghénane; ajustement du pH afin d'induire une coacervation complexe; adsorption du coacervat à la surface des particules d'arôme pour former des particules encapsulées; gélification du kappa-carraghénane en excès par réticulation ionique et formation des particules encapsulées avec deux écorces.

La gélification par réticulation ionique est effectué par addition de sels de calcium ou magnésium.

Dans ce contexte, les inventeurs de la présente invention ont mis au point de capsules à double parois pouvant contenir un actif lipophile dans les capsules primaires et éventuellement un actif hydrophile inclus dans la seconde avec un procédé de coacervation sans réticulation chimique.

Ces microcapsules originales sont constituées d'un cœur lipophile entouré d'une première couche de coacervat polymérique et d'une seconde couche comprenant un hydrogel. Elles possèdent de bonnes performances de contrainte à la rupture et se distinguent plus particulièrement par leur non toxicité puisque aucun agent de réticulation toxique n'est utilisé. Elles sont aussi modulables puisqu'on peut envisager d'encapsuler à la fois un actif lipophile dans le cœur et un actif hydrophile dans une matrice hydrogel. Elles peuvent être présentées soit sous forme humide soit sous forme sèche avec un prix de revient raisonnable.

Ainsi, l'invention a pour objet un procédé de préparation de capsules à double parois comprenant les étapes suivantes :
étape a) dispersion d'un principe actif lipophile dans une solution aqueuse, ladite solution contenant au moins un polymère anionique et au moins un polymère cationique ;
étape b) ajustement du pH de la solution obtenue à l'étape a) pour que les charges positives du polymère cationique équilibrent les charges négatives du polymère anionique afin d'induire une coacervation ;
étape c) adsorption des gouttelettes de coacervat issues de l'étape b) à la surface du principe actif pour former des capsules;
étape d) introduction d'une solution de polymères anioniques contenant un principe actif à encapsuler, hydrophile dans le milieu réactionnel contenant les capsules obtenues à l'étape c) ;
étape e) introduction du mélange issu de l'étape d) dans un moyen pour former des gouttes ;
étape f) mélange des gouttes issues de l'étape e) avec une solution de sels divalents et formation des capsules à double parois ;
caractérisé en ce qu'aucun agent réticulant autre que des sels divalents n'est utilisé.

Selon d'autres aspects particuliers, l'invention a pour objet :
- Un procédé tel que décrit ci-dessus, caractérisé en ce que ledit principe actif lipophile est choisi parmi les filtres solaires, les huiles essentielles, les vitamines A, C, D, E ou leurs dérivés, les parfums et arômes d'origine naturelle, les agents de bronzage ou de brunissement de la peau, les dérivés N-acylé d'acide aminé tels que le N-palmitoyl alanine, le N-palmitoyl glycine, le Npalmitoyl leucine, le N-palmitoyl isoleucine, le N-cocoyl alanine, le N-(ω-undecylenoyl) phenylalanine, les céramides, les phospholipides, les lipoaminoacides.
- Un procédé tel que décrit ci-dessus, caractérisé en ce que le polymère anionique est choisi parmi les polymères naturels comme la gomme arabique, les alginates, les carraghénates, les dérivés de la cellulose comme la carboxyméthyle cellulose, les dérivés d'amidon comme le carboxyméthyle amidon, ou les polymères synthétiques comme les polymères acryliques, méthacryliques, polylactiques, polyglycoliques ou leurs combinaisons.
- Un procédé tel que décrit ci-dessus, caractérisé en ce que le polymère cationique est choisi parmi les protéines animales comme la gélatine de porc ou de poisson, l'albumine, les protéines végétales issues par exemple du soja, de la pomme de terre ou du blé, le chitosan et ses dérivés, les polymères synthétiques issus de la combinaison d'amino acides comme la polylysine ou encore les polymères d'origine végétale comme la gomme guar et ses dérivés.
- Un procédé tel que décrit ci-dessus, caractérisé en ce que la solution de polymères anioniques de l'étape d) est une solution d'alginate de sodium.
- Un procédé tel que décrit ci-dessus, caractérisé en ce que ledit moyen pour former les gouttes mis en œuvre à l'étape e) est une buse ou une aiguille.
- Un procédé tel que décrit ci-dessus, caractérisé en ce que la solution de sels divalents de l'étape f) est choisie parmi les solutions de chlorure de calcium, de chlorure de baryum, de chlorure de manganèse.
- Un procédé tel que décrit ci-dessus, caractérisé en ce que la solution de polymères anioniques de l'étape d) contient optionnellement au moins un additif choisi parmi des solides insolubles finement divisés de nature minérale, comme par exemple des silices, des laponites, des alumino-silicates, du dioxyde de titane, du sulfate de calcium ou de nature organique comme des cires micronisées telles que la cire de carnauba ou la cire d'abeille, des polymères cationiques comme le chitosan ou la polylysine, de l'acide stéarique ou ses dérivés micronisés, de la cellulose microcristalline, des amidons.
- Un procédé tel que décrit ci-dessus comprenant l'étape g) de filtration des capsules obtenues à l'étape f) ; optionnellement une étape h) de lavage à l'eau ; et optionnellement une étape de séchage.

Les capsules peuvent enfin être séchées par tout procédé de séchage connu de l'homme de l'art, comme par exemple dans une étuve, un lyophilisateur ou un lit fluidisé. Elles peuvent également être remises en suspension dans une solution adéquate pour être stockées, transportées et utilisées sous forme liquide.

La divulgation concerne également une capsule à double parois comprenant un cœur lipophile entouré d'une première couche de coacervat polymérique et d'une seconde couche comprenant un hydrogel caractérisée en ce qu'elle ne contient aucune trace d'agent réticulant.

Selon d'autres aspects particuliers, la divulgation a pour objet :
- Une capsule telle que décrite ci-dessus caractérisée en ce que le cœur lipophile comprend un principe actif choisi parmi les filtres solaires, les huiles essentielles, les vitamines A, C, D, E ou leurs dérivés, les parfums et arômes d'origine naturelle, les agents de bronzage ou de brunissement de la peau, les dérivés N-acylé d'acide aminé tels que le N-palmitoyl alanine, le N-palmitoyl glycine, le Npalmitoyl leucine, le N-palmitoyl isoleucine, le N-cocoyl alanine, le N-(ω-undecylenoyl) phenylalanine, les céramides, les phospholipides, les lipoaminoacides.
- Une capsule telle que décrite ci-dessus, caractérisée en ce que l'hydrogel comprend un principe actif hydrophile choisi parmi des ingrédients montrant une action apaisante sur la peau comme l'allantoïne, le bisabolol, des ingrédients montrant une action hydratante de la peau comme par exemple l'urée, les hydroxy-urées, le glycérol, les polyglycérols, les glycéryl-glycosides et plus particulièrement le glycérol glucoside, les xylityl-glycosides et plus particulièrement le xylityl-glucoside, des extraits de polyphénols comme par exemple les extraits de polyphénols de raisin, les extraits de polyphénols de pin, les extraits de polyphénols de vin, les extraits de polyphénols d'olives, des extraits végétaux comme par exemple les extraits végétaux riches en tanins, les extraits végétaux riches en isoflavones, les extraits végétaux riches en terpènes, des extraits d'algues d'eau douce ou marines, des extraits bactériens, des ingrédients montrant une action antimicrobienne ou une action purifiante.
- Une capsule telle que décrite ci-dessus, ayant un diamètre compris entre 100 µm et 3000 µm et de préférence entre 500 µm et 2000 µm.
- Une capsule telle que décrite ci-dessus, susceptible d'être obtenue par le procédé tel que défini ci-dessus.
- Une capsule telle que décrite ci-dessus caractérisée en ce qu'elle comprend de 0,5% à 40 % en poids d'actif lipophile, plus particulièrement de 1% à 30%, et encore plus particulièrement de 1% à 20%, de 0% à 20% en poids d'actif hydrophile, plus particulièrement de 0,5% à 10% et encore plus particulièrement de 0,5% à 5%, de 0,1% à 5% en poids de polymère anionique, plus particulièrement de 0,5 % à 5%, et encore plus particulièrement de 1% à 3%.

La divulgation a enfin pour objet l'utilisation d'au moins une capsule telle que définie ci-dessus pour la préparation d'une composition cosmétique. Ainsi qu'une composition cosmétique comprenant de 0,01% à 20% massique, plus particulièrement de 1% à 10% massique d'au moins une capsule selon l'invention. En ce qui concerne les compositions cosmétiques comprenant les capsules, elles peuvent se présenter sous la forme d'émulsions H/E, E/H, E/H/E, H/E/H et/ou gels aqueux avec au moins un agent épaississant de phases hydrophiles.

Par ailleurs, les compositions cosmétiques se présentant sous la forme d'émulsions peuvent comprendre une phase huile épaissie par la présence de polymères épaississants d'huiles comme ceux décrits dans FR2961513A1 et dans FR2961210A1,

Les ingrédients encapsulés peuvent être des principes actifs cosmétiques comme :
a) des filtres solaires. Les filtres solaires peuvent être :
   1) des filtres solaires organiques choisis parmi les filtres organiques actifs dans l'UV-A, les filtres organiques actifs dans l'UV-B, les filtres organiques actifs dans l'UV-A et l'UV-B, et leurs mélanges.

Parmi les filtres organiques actifs dans l'UV-A, on peut citer :
- les dérivés du dibenzoylmethane, par exemple le ButylMethoxydibenzoylmethane ou Azobenzone commercialisé sous le nom de marque PARSOL™ 1789.
- le menthylanthranilate commercialisé sous le nom de marque NEO HELIOPAN™ MA par la société SYMRISE,
- leurs mélanges.

Parmi les filtres actifs seulement dans l'UV-B, on peut citer :
- les dérivés salicyliques, comme l'Homosalate commercialisé sous le nom de marque NEO HELIOPAN™ HMS; l'Ethylhexyl Salicylate commercialisé sous le nom de marque NEO HELIOPAN™ OS par SYMRISE; l'Octyl Salicylate commercialisé sous le nom de marque NEO HELIOPAN^{(R)} type 05;
- les cinnamates, comme par exemple commercialisé sous le nom de marque PARSOL™MCX par la société DSM, l'Isopropyl ethoxycinnamate, l'IsoamylMethoxycinnamate commercialisé sous le nom de marque NEO HELIOPAN™ E 1000 par SYMRISE, le DiisopropylMethylcinnamate, le Cinnoxate, le GlycerylEthylhexanoate- Dimethoxycinnamate;
- les dérivés du benzylidène camphre, comme par exemple le 3-Benzylidène, le Methylbenzylidène camphre commercialisé sous le nom de marque NEO HELIOPAN^{(R)} MBC;
- les dérivés de triazine, comme par exemple l'Ethylhexyltriazone commercialisé sous le nom de marque UVINUL™ T150 par BASF, le DiethylhexylButamidoTriazone commercialisé sous le nom de marque UVASORB™ HEB, le Bis- EthylhexyloxyphenolMethoxyphenyl- Triazine;
- les para-aminobenzoates (ou PABA), comme par exemple l'Ethyl PABA, l'EthylDihydroxypropyl PABA, l'EthylhexylDimethyl PABA commercialisé sous le nom de marque ESCALOL™ 507 par la société ISP ;
- les dérivés d'imidazolines, comme par exemple l'EthylhexylDimethoxybenzylideneDioxoimidazoline- Propionate;
- les dérivés du benzalmalonate comme par exemple les: polyorganosiloxanes comprenant une fonction benzalmalonate tels que le Polysilicone-15 commercialisé sous le nom de marque PARSOL^{(R)} SLX par la société DSM, le Di-neopentyl-4'- methoxybenzalmalonate;

Parmi les filtres actifs à la fois dans l'UV-A et dans l'UV-B, on peut citer :
- les dérivés de la benzophénone comme par exemple la Benzophénone-1 commercialisée sous le nom de marque UVINUL™ 400, la Benzophénone-2 commercialisée sous le nom de marque UVINUL™ D50, la Benzophénone-3 ou Oxybenzone commercialisée sous le nom de marque UVINUL™ M40, la Benzophénone-4 commercialisée sous le nom de marque UVINUL™ MS40, la Benzophénone-6 commercialisée sous le nom de marque HELISORB™ 11, la Benzophénone-8 commercialisée sous le nom de marque SPECTRASORB™ UV-24,
- les dérivés du phenylbenzotriazole, comme par exemple le DrometrizoleTrisiloxane commercialisé sous le nom de marque Silatrizole™ par la société RHODIA, le Méthylène bis- BenzotriazolylTetramethylbutylphenol commercialisé sous le nom de marque MIXXIM™ BB/100,
- les dérivés du bis-resorcinyltriazines, comme par exemple le Bis-EthylhexyloxyphenolMethoxyphenylTriazine,
- les dérivés de benzoxazole, comme par exemple le 2,4-bis-[5-l(dimethylpropyl)benzoxazol-2-yl-(4- phenyl)-imino]-6-(2-ethylhexyl)-imino-1,3,5-triazine commercialisé sous le nom de marque Uvasorb™ K2A par la société Sigma 3V;
- et leurs melanges.
   2) des filtres solaires inorganiques sont choisis parmi le talc, le kaolin, des pigments d'oxydes métalliques traités ou non comme par exemple des nanopigments d'oxyde de titane (amorphe ou cristallisé), de fer, de zinc, de zirconium ou de cérium.
   b) Des huiles essentielles, comme par exemple :
      ∘ les huiles essentielles riches en carbures terpéniques et sesquiterméniques, telles que l'huile essentielle de térébenthine, de genévrier, de citron,
      ∘ les huiles essentielles riches en alcools, telles que l'huile essentielle de coriandre, de rose, de bois de rose,
      ∘ les huiles essentielles riches en mélanges d'esters et d'alcools, telles que l'huile essentielle de lavande, de menthe,
      ∘ les huiles essentielles riches en aldéhydes, telles que les huiles essentielles de cannelle, de citronnelle, d'eucalyptus,
      ∘ les huiles essentielles riches en cétones, telles que les huiles essentielles de sauge, de camphrier, de thuya,
      ∘ les huiles essentielles riches en phénols, telles que les huiles essentielles de thym, de sarriette, d'origan, de girofle,
      ∘ les huiles essentielles riches en éthers, telles que les huiles essentielles de badiane, d'anis vert, de fenouil, d'eucalyptus globulus,
      ∘ les huiles essentielles riches en péroxydes, telles que les huiles essentielles de chénopode, d'ail,
      ∘ les huiles essentielles sulfurées, telles que les huiles essentielles de crucifères, de liliacées,
   c) Des substances parfumantes, comme par exemple :
      a. les huiles essentielles,
      b. les parfums et aromes d'origine naturelle, tels que les extraits de fleurs, de tiges, de feuilles, de fruits, de racines, de bois, d'aiguilles, de branches, d'herbes, de graminées, de résines, de baumes,
      c. les substances d'origine synthétiques telles que :
         i. des composés de type ester, comme par exemple l'acétate de benzyle, le benzoate de benzyle, l'isobutyrate de phénoxyéthyle, l'acétate de p-tert-butylcyclohexyle, l'acétate de citronéllyle, le formiate de citronéllyle, l'acétate de géranyle, l'acétate de linalyle, l'acétate de dimethyl-benzylcarbinyle, l'acétate de phenylethyle, le benzoate de linalyle, le formiate de benzyle, le glycinate d'ethylmethylphenyle, le propionate d'alkylcyclohexyle, le propionate de styralyle et le salicylate de benzyle,
         ii. des composés de type éther, comme par exemple le benzyléthyle éther,
         iii. des composés de type aldéhyde, comme par exemple le citral, le citronellal, le citronellyloxyacetaldehyde, le cyclamenaldehyde, l'hydroxycitronellal, le lilial et le bourgeonal,
         iv. des composés de type cétone, comme par exemple les ionones et plus particulièrement l'a-isométhylionone,
         v. des composés de type alcools aromatiques, comme par exemple l'anéthol, le citronellol, l'eugénol, l'isoeugénol, le géraniol, le linalol, le phényléthylalcool, le terpinéol,
         vi. les terpènes.
   d) Des agents de bronzage ou de brunissement de la peau, comme par exemple la dihydroxyacétone, l'isatinne, l'alloxane, la ninhydrine, l'érythrulose.
   e) Des colorants, comme par exemple le Yellow 5, l'Acid Blue 91, le Green 5, le Green 3 / Fast Green FCF 3, l'Orange 4, le Red 4 / Food Red 1, le Yellow 6, l'Acid Red 33 / Food Red 12, le Red 40, le carminé de cochenille (CI 15850, CI 75470), l'Ext. Violet 2, le Red 6-7, le Ferric Ferrocyanide, les Ultramarines, l'Acide Yellow 3 / Yellow 10, l'Acid Blue 3, le Yellow 10.
   f) Des vitamines ou leurs dérivés, comme par exemple le rétinol (vitamine A) et ses esters (palmitate de rétinyle par exemple), l'acide ascorbique (vitamine C) et ses esters, les dérivs de sucre de l'acide ascorbique (comme par exemple l'ascorbyl glucoside), le tocophérol (vitamine E) et ses esters (comme par exemple l'acétate de tocophérol), les vitamines B3 ou B10 (niacinamide et ses dérivés).
   g) Des acides aminés.
   h) Des hydrolysâts totaux ou partiels de protéines.
   i) Des dérivés N-acylés d'acides aminés de formule (A) : 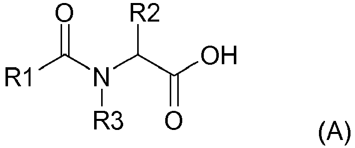 dans laquelle :
      - R1 représente un radical aliphatique, saturé ou insaturé, linéaire ou ramifié, comportant de 7 à 29 atomes de carbone,
      - R2 représente un atome d'hydrogène ou un radical choisi parmi les radicaux méthyle, isopropyle, isobutyle, 1-méthyl propyle, hydroxyméthyle, 1-hydroxy éthyle, thiométhyle, 2-méthylthio éthyle, 4-aminobutyle, 3-guanidino propyle, 3-uréido propyle, (1-amino carbonyl) méthyle, carboxy méthyle, 2-carboxy éthyle, 2-(amino carbonyl) éthyle, benzyle, 4-hydroxy benzyle, 3,4-dihydroxy benzyle, [1H-indol-3-yl]méthyle, (1H-imidazol-4-yl)méthyle, 3-amino propyle, et
      - R3 représente un atome d'hydrogène ou un radical méthyle.

Comme exemple de dérivés N-acylé d'acide aminé de formule (A), on peut citer le N-palmitoyl alanine, le N-palmitoyl glycine, le Npalmitoyl leucine, le N-palmitoyl isoleucine, le N-cocoyl alanine, le N-(w-undecylenoyl) phenylalanine ;
j) des dérivés N-acylés d'acides aminés de formule (B) 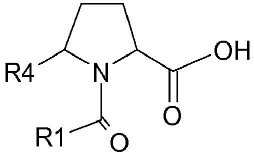 dans laquelle :
   - R1 représente un radical aliphatique, saturé ou insaturé, linéaire ou ramifié, comportant de 7 à 29 atomes de carbone, et
   - R4 représente un atome d'hydrogène ou un radical hydroxy.
k) Des ingrédients montrant une action éclaircissante et/ou blanchissante et/ou décolorante de la peau, comme par exemple l'arbutine, l'acide kojique, l'hydroquinone ou ses dérivés, le VEGEWHITE™, la GATULINE™, le SYNERLIGHT™, le BIOWHITE™, le PHYTOLIGHT™, le DERMALIGHT™, la CLARISKIN™, le MELASLOW™, le DERMAWHITE™, l'ETHIOLINE, le MELAREST™, le GIGAWHITE™, l'ALBATINE™, le LUMISKIN™.
l) Des ingrédients montrant une action apaisante sur la peau comme l'allantoïne, le bisabolol.
m) Des ingrédients montrant une action hydratante de la peau comme par exemple l'urée, les hydroxy-urées, le glycérol, les polyglycérols, les glycéryl-glycosides et plus particulièrement le glycérol glucoside, les xylityl-glycosides et plus particulièrement le xylityl-glucoside contenu dans la composition commercialisée sous le nom de marque AQUAXYL™.
n) Des extraits de polyphénols comme par exemple les extraits de polyphénols de raisin, les extraits de polyphénols de pin, les extraits de polyphénols de vin, les extraits de polyphénols d'olive.
o) Des extraits végétaux comme par exemple les extraits végétaux riches en tanins, les extraits végétaux riches en isoflavones, les extraits végétaux riches en terpènes,
p) des extraits d'algues d'eau douce ou marines.
q) des extraits bactériens.
r) des céramides.
s) des phospholipides.
t) des ingrédients montrant une action antimicrobienne ou une action purifiante, comme par exemple l'OCTOPIROX™ ou le SENSIVA™ SC50.

De manière surprenante, les microcapsules non réticulées obtenues à l'étape (d) sont stables en présence de la solution de polymères anioniques ajoutée et ne se brisent pas lorsqu'on réalise les étapes (e) et (f) du procédé selon l'invention. L'ingrédient lipophile reste confiné dans le cœur huileux de la nouvelle microcapsule et l'ingrédient hydrophile est encapsulé dans la seconde coquille d'alginate.

La solution de polymère anionique mise en œuvre à l'étape (e) peut contenir aussi des additifs technologiques destinés à renforcer la résistance mécanique des microbilles, à ajuster leur densité ou à moduler la cinétique de libération de l'ingrédient hydrophile. Ces additifs peuvent être des solides insolubles finement divisés de nature minérale, comme par exemple des silices, des laponites, des alumino - silicates, du dioxyde de titane, du sulfate de calcium ou de nature organique comme des cires micronisées (cire de carnauba, cires d'abeille...), des polymères cationiques comme le chitosan ou la polylysine, de l'acide stéarique ou ses dérivés micronisés, de la cellulose microcristalline, des amidons. Les additifs technologiques peuvent aussi être des produits solubles comme des sels minéraux, des glycols ou des tensioactifs permettant une meilleure dispersion des microcapsules ou facilitant les opérations de séchage.

Les exemples suivants décrivent une mise en œuvre du procédé selon l'invention et les microcapsules obtenues.

### EXEMPLE N°1

### Protocole de double encapsulation par coacervation avec protéine de pomme de terre et gomme arabique puis insertion dans des billes d'alginate/Laponite.

| **Matériel nécessaire** | **Produits nécessaires** |
|---|---|
| Réacteur en verre (2L) | 10g d'isolat de pomme de terre |
| Pale d'agitation | 15g de Gomme arabique |
| Bain chauffant | 625g d'eau déminéralisée |
| Béchers | 100g d'huile TCM (Triglycéride C8-C10) |
| Sonde à pH | 0,1g de colorant Red Oil |
| Agitateur magnétique | Acide acétique 10% |
| Filtre 100µm | 4g d'alginate en poudre |
| Seringue | 1g de Laponite |
| | Solution [CaCl₂]=4% |

### 1.1 Préparation de microcapsules par coacervation

### • Phase huile

0,1g de red oil et 100g d'huile TCM sont introduits dans un bécher et agités avec un agitateur magnétique pendant 20 minutes à 40°C. Filtrer et laisser refroidir.

### • Phase aqueuse

15g de gomme arabique, sont introduits dans un bécher contenant 400g d'eau. Agiter avec un agitateur magnétique jusqu'à dissolution (5 minutes) puis introduire le mélange dans le réacteur et agiter à 200 tours par minute (tpm).

### • Incorporation de la phase huile

Augmenter l'agitation à 350 tpm du réacteur puis introduire lentement l'huile. Laisser agiter pendant 15 minutes.

### • Isolat de pomme de terre

10g d'isolat de pomme de terre et 225g d'eau sont introduits dans un bécher et agités avec un agitateur magnétique. Lorsque la dissolution est complète (5 minutes), introduire très lentement la solution dans le réacteur en contrôlant le pH (environ 4 après tout l'ajout).

### • Abaissement du pH

Diminuer le pH du milieu à 3,65 avec de l'acide acétique à 10%. Pour que la coacervation se forme.

### • Augmentation de la température

Augmenter la température à 50°C pendant 1 heure pour durcir l'isolat de pomme de terre. Puis laisser refroidir et décanter.

### 1.2 Préparation de microbilles par double encapsulation

### • Préparation de la solution d'alginate

4g d'alginate et 1g de Laponite sont introduits lentement dans un bécher contenant 200g d'eau sous agitation vigoureuse pendant 30 minutes.

### • Mélange microcapsules/alginate

Peser 100g de microcapsules obtenues à l'étape 1.1 et les introduire dans 150g d'une solution à 2% d'alginate de sodium.

### • Formation des microbilles

Introduire le mélange dans une seringue et faire des gouttes dans la solution de chlorure de calcium. Laisser un temps de contact de 15 minutes. Pour finir, les rincer à l'eau.

### 1.3. Caractérisation des microbilles obtenues par le procédé.

Les billes non séchées obtenues par le procédé sont des sphères colorées ayant une taille moyenne de 1000 µm.

Elles contiennent environ 20% d'un cœur huileux, 5% d'un coacervat de gélatine/ gomme arabique, 1% d'alginate, 0,5% de laponite, le reste étant de l'eau.

Lorsqu'on exerce une pression mécanique sur les microbilles, elles éclatent en relargant de l'huile rouge.

### EXEMPLE N°2

### Protocole de double encapsulation par coacervation avec de la gélatine et gomme arabique puis insertion dans des billes d'alginate

### 2.1 Préparation des microcapsules par coacervation

| **Matériel nécessaire** | **Produits nécessaires** |
|---|---|
| Réacteur en verre (2L) | 12,5g de Gélatine |
| Pâle d'agitation | 12,5g de Gomme arabique |
| Bain chauffant | 550g d'eau déminéralisée |
| Bain de glace | 100g d'huile TCM |
| Béchers | 0,1g de colorant Red Oil |
| Sonde à pH | Acide acétique 10% |
| Thermomètre | 4g d'alginate en poudre |
| Agitateur magnétique | 1g de Laponite |
| Filtre 100µm | Solution [CaCl₂]=4% |
| Seringue | |

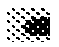 Phase huile

0,1g de red oil (utilisé comme ingrédient lipophile modèle à encapsuler) et 100g d'huile TCM sont introduits dans un bécher et agités avec un agitateur magnétique pendant 20 minutes à 40°C. Filtrer et laisser à 40°C.

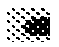 Phase aqueuse

300g d'eau sont introduits dans le réacteur thermostaté à 40°C.

12,5g de gomme arabique, 12,5g de gélatine sont introduits dans un bécher contenant 250g d'eau. Agiter à 40°C jusqu'à dissolution (15minutes). Puis ajouter le mélange dans le réacteur et agiter à 200 tpm.

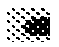 Incorporation de la phase huile

Augmenter l'agitation à 350 tpm puis introduire lentement l'huile chaude. Laisser agiter pendant 15 minutes.

### Abaissement du pH

Stopper la chauffe et diminuer le pH du milieu à 4,10 avec de l'acide acétique à 10%. Pour que la coacervation se forme.

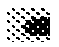 Abaissement de la température

Diminuer la température à 10°C pour durcir la gélatine. Laisser 15 minutes puis stopper l'agitation. Puis laisser décanter.

**2.2. Préparation de microbilles par double encapsulation**

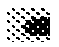 Préparation de la solution d'alginate

4g d'alginate et 1g de Laponite sont introduits lentement dans un bécher contenant 200g d'eau sous agitation vigoureuse pendant 30 minutes.

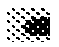 Mélange microcapsules/alginate (50/50)

Peser 100g de microcapsules et les introduire dans la même quantité de la solution d'alginate de sodium. Homogénéiser sous agitation magnétique.

Introduire le mélange dans une seringue et faire des gouttes dans la solution de chlorure de calcium. Laisser un temps de contact de 15 minutes, puis les rincer à l'eau.

### 2.3. Caractérisation des microbilles obtenues par le procédé.

Les microbilles obtenues sont translucides, de taille moyenne environ 800µm. Les microcapsules primaires d'huile et de gélatine sont clairement visibles à l'intérieur des microbilles.

Les microbilles contiennent environ 20% d'un cœur huileux, 5% d'un coacervat de gélatine / gomme arabique, 1% d'alginate, 0,5% de laponite, le reste étant de l'eau.

Il a été vérifié que les microbilles obtenues selon l'invention sont stables et étanches. Pour cela, elles ont été dispersées dans une huile minérale et soumise à agitation magnétique pendant deux heures. La coloration de l'huile minérale a été observée au bout de deux heures. Toute coloration de cette huile traduit une diffusion du colorant ou une rupture des microcapsules. A titre de témoin, des microcapsules réalisées par le procédé de coacervation sans réticulation obtenues telles que décrit au paragraphe 2.1 et des microbilles obtenues par le même procédé mais réticulées avec du glutaraldéhyde ont été soumises au même test.

| | Microcapsules non réticulées | Microcapsules réticulées au glutaraldéhyde | Microbilles selon l'invention |
|---|---|---|---|
| Coloration de l'huile après 2 h | Rouge vif | Aucune coloration | Aucune coloration |

Ces résultats démontrent la bonne stabilité des microbilles obtenues selon l'invention.

### EXEMPLE N°3

### Protocole de double encapsulation des microcapsules (par coacervation avec de la gélatine) dans des billes d'alginate.

### 3.1 Préparation des microcapsules par coacervation

| **Matériel nécessaire** | **Produits nécessaires** |
|---|---|
| Réacteur en verre (2L) | 12,5g de Gélatine |
| Pâle d'agitation | 12,5g de Gomme arabique |
| Bain chauffant | 550g d'eau déminéralisée |
| Bain de glace | 100g d'huile TCM |
| Béchers | 0,1g de colorant Red Oil |
| Sonde à pH | Acide acétique 10% |
| Thermomètre | 4g d'alginate en poudre |
| Agitateur magnétique | 1g de caféine |
| Filtre 100µm | Solution [CaCl₂]=4% |
| Générateur de gouttes NISCO VAR D | |

Le même protocole que celui décrit à l'exemple 2 est mis en œuvre.

**3.2 Préparation de microbilles par double encapsulation**

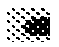 Préparation de la solution d'alginate et de caféine

4g d'alginate et 1g de caféine sont introduits lentement dans un bécher contenant 200g d'eau sous agitation vigoureuse pendant 30 minutes.

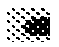 Mélange microcapsules/alginates (50/50)

Peser 100g de microbilles obtenues à l'étape 3.1, et y introduire la même quantité d'alginate à 2%. Mélanger pour obtenir une suspension homogène.

Introduire le mélange dans le réservoir du générateur de gouttes NISCO VAR D équipé d'une buse de diamètre 800µm et mettre en marche l'appareil avec les paramètres suivants :
Débit: 12ml/min, fréquence des vibrations de la buse vibrante: 0,22khz, amplitude 79%.

Les gouttes générées sont récoltées dans la solution de chlorure de calcium où elles forment des microbilles solides. Laisser un temps de contact de 15 minutes, filtrer les billes et les rincer à l'eau.

### 3.3. Caractérisation des microbilles obtenues par le procédé

On obtient des microbilles de diamètre moyen 1500µm, contenant deux ingrédients encapsulés : la caféine dans la matrice externe d'alginate et le Red oil dans le cœur huileux. Leur composition quantitative est d'environ 20% d'huile, 5% d'un coacervat de gélatine / gomme arabique, 1% d'alginate, 0,5% de caféine, le reste étant de l'eau.

## Revendications

1. Procédé de préparation de capsules à double parois comprenant les étapes suivantes :
étape a) dispersion d'un principe actif lipophile dans une solution aqueuse, ladite solution contenant au moins un polymère anionique et au moins un polymère cationique ;
étape b) ajustement du pH de la solution obtenue à l'étape a) pour que les charges positives du polymère cationique équilibrent les charges négatives du polymère anionique afin d'induire une coacervation ;
étape c) adsorption des gouttelettes de coacervat issues de l'étape b) à la surface du principe actif pour former des capsules;
étape d) introduction d'une solution de polymères anioniques contenant un principe actif à encapsuler, hydrophile dans le milieu réactionnel contenant les capsules obtenues à l'étape c) ;
étape e) introduction du mélange issu de l'étape d) dans un moyen pour former des gouttes ;
étape f) mélange des gouttes issues de l'étape e) avec une solution de sels divalents et formation des capsules à double parois ;
**caractérisé en ce qu'**aucun agent réticulant autre que des sels divalents n'est utilisé.

2. Procédé selon la revendication 1, **caractérisé en ce que** ledit principe actif lipophile est choisi parmi les filtres solaires, les huiles essentielles, les vitamines A, C, D, E ou leurs dérivés, les parfums et arômes d'origine naturelle, les agents de bronzage ou de brunissement de la peau, les dérivés N-acylé d'acide aminé tels que le N-palmitoyl alanine, le N-palmitoyl glycine, le Npalmitoyl leucine, le N-palmitoyl isoleucine, le N-cocoyl alanine, le N-(ω-undecylenoyl) phenylalanine, les céramides, les phospholipides, les lipoaminoacides.

3. Procédé selon l'une des revendications 1 et 2, **caractérisé en ce que** le polymère anionique est choisi parmi les polymères naturels comme la gomme arabique, les alginates, les carraghénates, les dérivés de la cellulose comme la carboxyméthyl cellulose, les dérivés d'amidon comme le carboxyméthyl amidon, ou les polymères synthétiques comme les polymères acryliques, méthacryliques, polylactiques, polyglycoliques ou leurs combinaisons.

4. Procédé selon l'une des revendications 1 à 3, **caractérisé en ce que** le polymère cationique est choisi parmi les protéines animales comme la gélatine de porc ou de poisson, l'albumine, les protéines végétales issues par exemple du soja, de la pomme de terre ou du blé, le chitosan et ses dérivés, les polymères synthétiques issus de la combinaison d'amino acides comme la polylysine ou encore les polymères d'origine végétale comme la gomme guar et ses dérivés.

5. Procédé selon l'une des revendications 1 à 4, **caractérisé en ce que** la solution de polymères anioniques de l'étape d) est une solution d'alginate de sodium.

6. Procédé selon l'une des revendications 1 à 5, **caractérisé en ce que** ledit moyen pour former les gouttes mis en œuvre à l'étape e) est une buse ou une aiguille.

7. Procédé selon l'une des revendications 1 à 6, **caractérisé en ce que** la solution de sels divalents de l'étape f) est choisie parmi les solutions de chlorure de calcium, de chlorure de baryum, de chlorure de manganèse.

8. Procédé selon l'une des revendications 1 à 7, caractérisé en ce la solution de polymères anioniques de l'étape d) contient au moins un additif choisi parmi des solides insolubles finement divisés de nature minérale, comme par exemple des silices, des laponites, des alumino-silicates, du dioxyde de titane, du sulfate de calcium ou de nature organique comme des cires micronisées telles que la cire de carnauba ou la cire d'abeille, des polymères cationiques comme le chitosan ou la polylysine, de l'acide stéarique ou ses dérivés micronisés, de la cellulose microcristalline, des amidons.

9. Procédé selon l'une des revendications 1 à 8 comprenant l'étape g) de filtration des capsules obtenues à l'étape f) ; optionnellement une étape h) de lavage à l'eau ; et optionnellement une étape de séchage.

## Patentansprüche

1. Verfahren zur Zubereitung von doppelwandigen Kapseln, umfassend die folgenden Schritte:
Schritt a) Dispersion eines lipophilen Wirkstoffs in einer wässrigen Lösung, wobei die Lösung mindestens ein anionisches Polymer und mindestens ein kationisches Polymer enthält;
Schritt b) Einstellung des pH-Werts der in Schritt a) erhaltenen Lösung, damit die positiven Ladungen des kationischen Polymers die negativen Ladungen des anionischen Polymers ausgleichen, um eine Koazervation einzuführen;
Schritt c) Adsorption von aus Schritt b) stammenden Koazervattröpfchen an der Oberfläche des Wirkstoffs, um Kapseln zu bilden;
Schritt d) Einleitung einer Lösung anionischer Polymere, die einen einzukapselnden Wirkstoff enthalten, hydrophil in dem Reaktionsmedium, das die in Schritt c) erhaltenen Kapseln enthält;
Schritt e) Einleitung der aus Schritt d) stammenden Mischung in ein Mittel zum Bilden von Tropfen;
Schritt f) Mischen der aus Schritt e) stammenden Tropfen mit einer Lösung zweiwertiger Salze und Bilden der doppelwandigen Kapseln;
**dadurch gekennzeichnet, dass** kein anderes Vernetzungsmittel als die zweiwertigen Salze verwendet wird.

2. Verfahren nach Anspruch 1, **dadurch gekennzeichnet, dass** der lipophile Wirkstoff ausgewählt ist aus den Sonnenschutzmitteln, den ätherischen Ölen, den Vitaminen A, C, D, E oder deren Derivaten, den Parfums und Aromen natürlichen Ursprungs, den Bräunungs- oder Hautbräunungsmitteln, N-acylierten Derivaten von Aminosäure, wie N-Palmitoyl Alanin, N-Palmitoyl Glycin, NPalmitoyl Leucin, N-Palmitoyl Isoleucin, N-Cocoyl Alanin, N-(ω-Undecylenoyl)phenylalanin, Ceramiden, Phospholipiden, Lipoaminosäuren.

3. Verfahren nach einem der Ansprüche 1 und 2, **dadurch gekennzeichnet, dass** das anionische Polymer ausgewählt ist aus natürlichen Polymeren wie dem Gummi arabicum, den Alginaten, den Carrageenaten, den Cellulosederivaten wie Carboxymethylcellulose, Amidonderivaten wie Carboxymethylamidon oder den synthetischen Polymeren wie den Acryl-, Methacryl- Polymilchsäure-, Polyglycolpolymeren oder deren Kombinationen.

4. Verfahren nach einem der Ansprüche 1 bis 3, **dadurch gekennzeichnet, dass** das kationische Polymer ausgewählt ist aus den tierischen Proteinen wie der Schweine- oder Fischgelatine, dem Albumin, den pflanzlichen Proteinen, die zum Beispiel von Soja, Kartoffel oder Weizen stammen, dem Chitosan und seinen Derivaten, den synthetischen Polymeren, die aus der Kombination von Aminosäuren stammen, wie Polylysin oder auch die Polymere pflanzlichen Ursprungs wie der Guargummi und dessen Derivate.

5. Verfahren nach einem der Ansprüche 1 bis 4, **dadurch gekennzeichnet, dass** die Lösung von anionischen Polymeren aus Schritt d) eine Lösung aus Natriumalginat ist.

6. Verfahren nach einem der Ansprüche 1 bis 5, **dadurch gekennzeichnet, dass** das in Schritt e) eingesetzte Mittel zum Bilden von Tropfen eine Düse oder eine Nadel ist.

7. Verfahren nach einem der Ansprüche 1 bis 6, **dadurch gekennzeichnet, dass** die Lösung zweiwertiger Salze aus Schritt f) ausgewählt ist aus den Lösungen von Calciumchlorid, Bariumchlorid und Manganchlorid.

8. Verfahren nach einem der Ansprüche 1 bis 7, **dadurch gekennzeichnet, dass** die Lösung von anionischen Polymeren aus Schritt d) mindestens einen Zusatzstoff enthält, ausgewählt aus feinteiligen unlöslichen Feststoffen mineralischer Art, wie zum Beispiel Siliciumdioxide, Laponite, Aluminosilikate, Titandioxid, Calciumsulfat, oder organischer Art, wie mikronisierte Wachse wie Carnaubawachs oder Bienenwachs, kationischen Polymeren wie das Chitosan oder das Polylysin, Stearinsäure oder deren mikronisierten Derivaten, mikrokristalliner Cellulose, Amidonen.

9. Verfahren nach einem der Ansprüche 1 bis 8, umfassend den Schritt g) des Filterns der in Schritt f) erhaltenen Kapseln; wahlweise einen Schritt h) des Waschens mit Wasser; und wahlweise einen Trocknungsschritt.

## Claims

1. Method for preparing double-walled capsules comprising the following steps:
step a) dispersing a main lipophilic agent in an aqueous solution, said solution containing at least one anionic polymer and at least one cationic polymer;
step b) adjusting the pH of the solution obtained in step a) such that the positive charges of the cationic polymer balance the negative charges of the anionic polymer in order to induce a coacervation;
step c) adsorbing coacervate droplets coming from step b) to the surface of the main agent to form capsules;
step d) introducing an anionic polymer solution containing a main agent to be encapsulated, hydrophilic in the reactional environment containing the capsules obtained in step c);
step e) introducing the mixture coming from step d) in a means for forming drops;
step f) mixing drops coming from step e) with a divalent salt solution and forming double-walled capsules;
**characterised in that** no cross-linking agent, other than divalent salts, is used.

2. Method according to claim 1, **characterised in that** said main lipophilic agent is selected from among solar filters, essential oils, vitamins A, C, D, E or the derivatives thereof, scents and aromas of natural origin, agents for tanning or browning the skin, N-acyl amino acid derivatives, such as N-palmitoyl alanine, N-palmitoyl glycine, N-palmitoyl leucine, N-palmitoyl isoleucine, N-cocoyl alanine, N-(ω-undecylenoyl) phenylalanine, ceramides, phospholipids, lipoamino acids.

3. Method according to one of claims 1 and 2, **characterised in that** the anionic polymer is selected from among natural polymers such as acacia gum, alginates, carrageenans, cellulose derivates such as carboxymethyl cellulose, starch derivatives such as carboxymethyl starch, or synthetic polymers such as acrylic, methacrylic, polylactic, polyglycolic polymers, or the combinations thereof.

4. Method according to one of claims 1 to 3, **characterised in that** the cationic polymer is selected from among animal proteins such as pork or fish gelatine, albumin, plant proteins coming from, for example, soya, potato or wheat, chitosan and the derivatives thereof, synthetic polymers coming from the combination of amino acids such as polylysine or also polymers of plant origin, such as guar gum and the derivatives thereof.

5. Method according to one of claims 1 to 4, **characterised in that** the anionic polymer solution of step d) is a sodium alginate solution.

6. Method according to one of claims 1 to 5, **characterised in that** said means for forming drops implemented in step e) is a nozzle or a needle.

7. Method according to one of claims 1 to 6, **characterised in that** the divalent salt solution of step f) is selected from among calcium chloride, barium chloride, manganese chloride solutions.

8. Method according to one of claims 1 to 7, **characterised in that** the anionic polymer solution of step d) contains at least one additive selected from among finely split insoluble solids of mineral nature, such as for example silicas, laponites, alumino-silicates, titanium dioxide, calcium sulphate or of organic nature, such as micronised waxes such as carnauba wax or beeswax, stearic acid or the micronised derivatives thereof, microcrystalline cellulose, starches.

9. Method according to one of claims 1 to 8 comprising step g) of filtering capsules obtained in step f); optionally a step h) of washing with water; and optionally a drying step.
